# EUROPÄISCHE PATENTSCHRIFT

(11) **EP 0 661 058 B1**
(45) Veröffentlichungstag und Bekanntmachung des Hinweises auf die Patenterteilung: **03.12.2003**
(21) Anmeldenummer: 94120774.8
(22) Anmeldetag: 27.12.1994
(51) Int. Cl.: A61K 38/08, A61K 31/52, A61P 31/12, A61P 31/22

(54) **Verwendung von Bradykinin-Antagonisten zur Behandlung von Viruserkrankungen**
Use of bradykinin antagonists for the treatment of viral diseases
Utilisation des antagonistes de bradykinine pour le traitement des maladies virales

(30) Priorität: 31.12.1993 DE 4345062
(43) Veröffentlichungstag der Anmeldung: 05.07.1995
(73) Patentinhaber: Aventis Pharma Deutschland GmbH, 65929 Frankfurt am Main (DE)
(72) Erfinder: Lembeck, Fred, Prof. Dr., A-8042 Graz (AT); Wirth, Klaus, Dr., D-65830 Kriftel (DE); Winkler, Irvin, Dr., D-65835 Liederbach (DE); Breipohl, Gerhard, Dr., D-60529 Frankfurt (DE); Henke, Stephan, Dr., D-65719 Hofheim (DE); Knolle, Jochen, Dr., D-65830 Kriftel (DE)

(56) Entgegenhaltungen:
- EP-A- 0 370 453
- J. CELL. BIOCHEM., Bd. 14c,Nr. supplement, 1990 Seite 226 J.M. STEWART ET AL. 'Bradykinin antagonists: design and applications'
- DRUGS OF THE FUTURE, Bd. 17,Nr. 4, 1992 D.J. KYLE ET AL. 'Recent advances toward novel bradykinin antagonists.'
- ANTIVIRAL RES., Bd. 14,Nr. 6, 1990 Seiten 339-344, P.G. HIGGINS ET AL. 'A study of the efficacy of the bradykinin antagonist NPC 567 in rhinovirus infections in human volunteers.'
- EUR. RESPIR. J., Bd. 6,Nr. 4, 1993 Seiten 576-587, A. TRIFILIEFF ET AL. 'Kinins and respiratory tract diseases.'

## Beschreibung

Die Erfindung betrifft die Verwendung von Bradykinin-Antagonisten in Kombination mit mindestens einem weiteren Antivirusmittel zur Herstellung von Arzneimitteln zur Behandlung von Erkrankungen, die durch Herpesviren oder Varicella Zoster Viren hervorgerufen werden.

Bradykinin und verwandte Peptide sind potente Entzündungen und Schmerzen erzeugende und vasoaktive körpereigene Substanzen. Die Verwendung von Bradykinin-Antagonisten als Mittel zur Bekämpfung von Zuständen, die durch Bradykinin vermittelt, ausgelöst oder unterstützt werden, ist bekannt (EP 0370453).

Überraschenderweise wurde nun gefunden, daß Bradykinin-Antagonisten der Formel I in Kombination mit mindestens einem weiteren Antivirusmittel geeignete Mittel zur Behandlung von Erkrankungen sind, die durch Herpesviren oder Varicella Zoster Viren hervorgerufen werden, wobei die Formel I wie folgt lautet

Z - P - A - B - C - E - F - K - (D)Q - G - M - F' - I (I),

in welcher bedeuten:
- Z: a₁) Wasserstoff, (C₁-C₈)-Alkyl, (C₁-C₈)-Alkanoyl, (C₁-C₈)-Alkoxycarbonyl, (C₃-C₈)-Cycloalkyl, (C₄-C₉)-Cycloalkanoyl, oder (C₁-C₈)-Alkylsulfonyl,
in welchen jeweils 1, 2 oder 3 Wasserstoffatome gegebenenfalls durch 1, 2 oder 3 gleiche oder verschiedene Reste aus der Reihe
Carboxy, NHR(1), [(C₁-C₄)-Alkyl]NR(1) oder [(C₆-C₁₀)-Aryl-(C₁-C₄)-alkyl]NR(1),
wobei R(1) für Wasserstoff oder eine Urethan-Schutzgruppe steht,
(C₁-C₄)-Alkyl, (C₁-C₈)-Alkylamino, (C₆-C₁₀)-Aryl-(C₁-C₄)-alkylamino, Hydroxy, (C₁-C₄)-Alkoxy, Halogen, Di-(C₁-C₈)-alkylamino, Di-[(C₆-C₁₀)-aryl-(C₁-C₄)]-alkylamino, Carbamoyl, Phthalimido, 1,8-Naphthalimido, Sulfamoyl, (C₁-C₄)-Alkoxycarbonyl, (C₆-C₁₄)-Aryl und (C₆-C₁₄)-Aryl-(C₁-C₅)-alkyl ersetzt sind,
oder in welchen jeweils 1 Wasserstoffatom gegebenenfalls durch einen Rest aus der Reihe
(C₃-C₈)-Cycloalkyl, (C₁-C₆)-Alkylsulfonyl, (C₁-C₆)-Alkylsulfinyl, (C₆-C₁₄)-Aryl-(C₁-C₄)-alkylsulfonyl, (C₆-C₁₄)-Aryl-(C₁-C₄)-alkylsulfinyl, (C₆-C₁₄)-Aryl, (C₆-C₁₄)-Aryloxy, (C₃-C₁₃)-Heteroaryl und (C₃-C₁₃)-Heteroaryloxy
und 1 oder 2 Wasserstoffatome durch 1 oder 2 gleiche oder verschiedene Reste aus der Reihe
Carboxy, Amino, (C₁-C₈)-Alkylamino, Hydroxy, (C₁-C₄)-Alkoxy, Halogen, Di-(C₁-C₈)-alkylamino, Carbamoyl, Sulfamoyl, (C₁-C₄)-Alkoxycarbonyl, (C₆-C₁₄)-Aryl und (C₆-C₁₄)-Aryl-(C₁-C₅)-alkyl ersetzt sind;
a₂) (C₆-C₁₄)-Aryl, (C₇-C₁₅)-Aroyl, (C₆-C₁₄)-Arylsulfonyl, (C₃-C₁₃)-Heteroaryl, oder (C₃-C₁₃)-Heteroaroyl;
a₃) Carbamoyl, das gegebenenfalls am Stickstoff durch (C₁-C₈)-Alkyl, (C₆-C₁₄)-Aryl oder (C₆-C₁₄)-Aryl-(C₁-C₅)-alkyl substituiert sein kann;
wobei in den unter a₁), a₂) und a₃) definierten Resten die Aryl-, Heteroaryl-, Aroyl-, Arylsulfonyl- und Heteroaroyl-Gruppen gegebenenfalls durch 1, 2, 3 oder 4 Reste aus der Reihe
Carboxy, Amino, Nitro, (C₁-C₈)-Alkylamino, Hydroxy, (C₁-C₆)-Alkyl, (C₁-C₆)-Alkoxy, (C₆-C₁₄)-Aryl, (C₇-C₁₅)-Aroyl, Halogen, Cyano, Di-(C₁-C₈)-alkylamino, Carbamoyl, Sulfamoyl und (C₁-C₆)-Alkoxycarbonyl substituiert sind;
- P: eine direkte Bindung oder einen Rest der Formel II,

- NR(2) -(U)- CO - (II)
worin
R(2) Wasserstoff, Methyl oder eine Urethan-Schutzgruppe bedeutet,
- U: (C₃-C₈)-Cycloalkyliden, (C₆-C₁₄)-Aryliden, (C₃-C₁₃)-Heteroaryliden, (C₆-C₁₄)-Aryl-(C₁-C₆)-alkyliden, welche gegebenenfalls substituiert sein können, oder [CHR(3)]ₙ bedeutet,
wobei n 1 - 8, vorzugsweise 1 - 6 ist, R(3) unabhängig voneinander Wasserstoff, (C₁-C₆)-Alkyl, (C₃-C₈)-Cycloalkyl, (C₆-C₁₄)-Aryl, (C₃-C₁₃)-Heteroaryl, bedeutet, die mit Ausnahme des Wasserstoffs jeweils gegebenenfalls durch
Amino, substituiertes Amino, Amidino, substituiertes Amidino, Hydroxy, Carboxy, Carbamoyl, Guanidino, substituiertes Guanidino, Ureido, substituiertes Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 1,8-Naphthalimido, 4-Imidazolyl, 3-Indolyl, 2-Thienyl, 3-Thienyl, 2-Pyridyl, 3-Pyridyl oder Cyclohexyl monosubstituiert sind,
wobei substituiertes Amino bevorzugt für -N(A')-Z, substituiertes Amidino bevorzugt für -(NH = )C-NH-Z, substituiertes Guanidino bevorzugt für -N(A')-C[=N(A')]-NH-Z und substituiertes Ureido bevorzugt für -CO-N(A')-Z stehen, in denen A' unabhängig voneinander Wasserstoff oder Z bedeutet, wobei Z wie unter a₁) oder a₂) definiert ist;
oder
worin R(2) und R(3) zusammen mit den diese tragenden Atomen ein mono-, bi- oder tricyclisches Ringsystem mit 2 bis 15 C-Atomen bilden;

- A: wie P definiert ist;
- B: eine basische Aminosäure in der L- oder D-Konfiguration, die in der Seitenkette substituiert sein kann;
- C: eine Verbindung der Formel III a oder III b

G'-G'-Gly (III a)

G'-NH-(CH₂)ₚ-CO (III b)

worin
p 2 bis 8 ist, und
G' unabhängig voneinander einen Rest der Formel IV

- NR(4)-CHR(5)-CO- (IV)

worin
R(4) und R(5) zusammen mit den diese tragenden Atomen ein heterocyclisches mono-, bi- oder tricyclisches Ringsystem mit 2 bis 15 C-Atomen bilden;
- E: den Rest einer neutralen, sauren oder basischen, aliphatischen oder alicyclisch-aliphatischen Aminosäure,
- F: unabhängig voneinander den Rest einer neutralen, sauren oder basischen, aliphatischen oder aromatischen Aminosäure, die in der Seitenkette substituiert sein kann, oder eine direkte Bindung;
- (D)Q: D-Tic, D-Phe, D-Dic, D-Thi oder D-Nal, die gegebenenfalls durch Halogen, Methyl oder Methoxy substituiert sein können oder einen Rest der nachstehenden Formel (V) 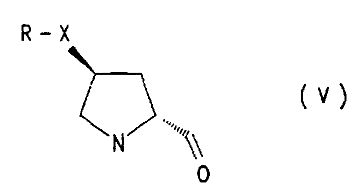 worin
X für Sauerstoff, Schwefel oder eine direkte Bindung steht;
R Wasserstoff, (C₁-C₈)-Alkyl, (C₃-C₈)-Cycloalkyl, (C₆-C₁₄)-Aryl, (C₆-C₁₄)-Aryl-(C₁-C₄)-alkyl, wobei der Alicyclus gegebenenfalls durch Halogen, Methyl oder Methoxy substituiert sein kann;
- G: wie G' oben definiert ist oder eine direkte Bindung;
- F': wie F definiert ist, einen Rest -NH-(CH₂)_{q}-, mit q = 2 bis 8, oder, falls G keine direkte Bindung bedeutet, eine direkte Bindung;
- I: -OH, -NH₂ oder NHC₂H₅;
- K: den Rest -NH-(CH₂)ₓ-CO- mit x = 1 - 4 oder eine direkte Bindung, und
- M: wie F definiert ist,
sowie deren physiologisch verträgliche Salze.

Geeignete Bradykinin-Antagonisten sind zum Beispiel beschrieben in den Patent-Veröffentlichungen EP 370 453, EP 472 220, WO 92/18155, WO 92/18156 und WO 92/17201 [Cortech; Bradykinin-Antagonisten der Formel X(BKA)ₙ, worin X ein Bindeglied, BKA die Peptidkette eines Bradykinin-Antagonisten und n eine ganze Zahl größer als 1 ist; Bradykinin-Antagonisten der Formel X(BKA); und Bradykinin-Antagonisten der Formel (Y)(X)(BKA) mit Y gleich einem Liganden, welcher ein Antagonist oder ein Agonist für einen Nicht-Bradykinin-Rezeptor ist].

Besonders geeignet sind Peptide der Formel I, in welcher bedeuten:
- Z: Wasserstoff oder wie unter a₁), a₂) oder a₃) definiert,
- P: eine Bindung oder ein Rest der Formel 11

- NR(2) -(U)- CO - (II)

mit
U gleich CHR(3) und
R(3) wie oben defininert,
R(2) gleich H oder CH₃,
- A: eine Bindung.

Insbesondere sind Verbindungen der Formel I bevorzugt, in der bedeuten:
- Z: Wasserstoff oder wie unter a₁), a₂) oder a₃) definiert,
- P: eine Bindung oder ein Rest der Formel II

- NR(2) -(U)- CO - (II)

mit
U gleich CHR(3) und
mit R(3) unabhängig voneinander Wasserstoff, (C₁-C₆)-Alkyl, (C₃-C₈)-Cycloalkyl, (C₆-C₁₄)-Aryl, (C₃-C₁₃)-Heteroaryl, die mit Ausnahme des Wasserstoffs jeweils gegebenenfalls durch
Amino, substituiertes Amino, Hydroxy, Carboxy, Carbamoyl, Guanidino, substituiertes Guanidino, Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 4-Imidazolyl, 3-Indolyl, 2-Thienyl, 3-Thienyl, 2-Pyridyl, 3-Pyridyl oder Cyclohexyl monosubstituiert sind,
wobei substituiertes Amino bevorzugt für -N(A')-Z und substituiertes Guanidino bevorzugt für -N(A')-C[=N(A')]-NH-Z stehen, in denen A' unabhängig voneinander Wasserstoff oder Z bedeutet, wobei Z wie unter a₁) oder a₂) definiert ist;
oder
worin R(2) und R(3) zusammen mit den diese tragenden Atomen ein mono-, bi- oder tricyclisches Ringsystem mit 2 bis 15 C-Atomen bilden;
R(2) gleich H oder CH₃,
- A: eine Bindung,
- (D)Q: D-Tic.

Ganz besonders geeignet ist (R)-Arginyl-(S)-arginyl-(S)-prolyl-(2S,4R)-hydroxyprolyl)glycyl-(S)-[3-(2-thienyl)alanyl]-(S)-seryl-(R)-[(1,2,3,4-tetrahydro-3-isochinolyl)carbonyl]-(2S,3aS,7aS)-[(hexahydro-2-indolinyl)carbonyl]-(S)-arginin, N-Acetat, das den INN-Namen Icatibant-Acetat trägt und auch als HOE 140 bezeichnet wird.

Erfindungsgemäß einsetzbar sind unterschiedliche weitere Antivirusmittel, wie z.B., Acyclovir, Val-Acyclovir, Pencyclovir, BVA-Uracil, Vidarabin, Ioddesoxyuridin, Broravir, Zidovudin (AZT), Didanosin (DDI), Dideoxycytidin (DDC) und Lamivudin (3-TC), insbesondere Acyclovir. Die genannten Verbindungen sind käuflich oder lassen sich nach allgemein bekannten Verfahren herstellen (vgl. Merck-Index, 11. Auflage Rahway, N.J. 1989, Drugs 45 (4), 488 ff., 45 (5), 637 ff., 1993).

Ein besonders bevorzugtes Kombinationspräparat zur Verwendung gemäß Anspruch 1 enthält Hoe 140 und Acyclovir.

Erfindungsgemäß einsetzbar sind die obenerwähnten Kombinationspräparate zur Herstellung eines Medikaments zur Bekämpfung von Herpesviren (z.B. HSV-1, HSV-2, HSV-3, VSV) und bei der Rekurrenz von Varicella Zoster Viren (VSV).

Die genannten Bradykinin-Antagonisten in Kombination mit mindestens einem weiteren Antivirusmittel dienen in geeigneter Verabreichungsform als Medikamente zur Behandlung von Viruserkrankungen gemäß Anspruch 1.

Geeignete pharmazeutische Präparate enthalten eine wirksame Menge des Bradykinin-Antagonisten - einzeln oder in Kombination - zusammen mit einem anorganischen oder organischen pharmazeutisch verwendbaren Trägerstoff und ggf. zusammen mit einem oder mehreren weiteren Antivirusmitteln.

Die Anwendung kann enteral, parenteral - wie z. B. subkutan, i. m. oder i. v. -, sublingual, epikutan, nasal, rektal, intravaginal, intrabukkal oder per Inhalation erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für die orale Anwendungsform oder zur Applikation auf die Schleimhäute werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trockenund Feuchtgranulat erfolgen. Als ölige Trägerstoffe oder Lösungsmittel kommen beispielsweise pflanzliche oder tierische Öle in Betracht, wie Sonnenblumenöl und Lebertran.

Ein Präparat für die topische Anwendung kann als wäßrige oder ölige Lösung, Lotion, Emulsion oder Gelee, Salbe oder Fettsalbe oder, falls möglich, in Sprayform vorliegen, wobei gegebenenfalls durch Zusatz eines Polymers die Haftung verbessert werden kann.

Für die intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Wäßrigen intranasalen Zubereitungen können Chelatbildner, Ethylendiamin-N,N,N',N'-tetraessigsäure, Citronensäure, Weinsäure oder deren Salze zugefügt werden. Die Applikation der Nasallösungen kann mittels Dosierzerstäuber erfolgen oder als Nasaltropfen mit viskositätserhöhendem Anteil bzw. Nasengels oder Nasencremes.

Für die inhalative Anwendung können Vernebler oder Druckgas-Packungen unter Verwendung inerter Trägergase benutzt werden.

Zur intravenösen, subkutanen, epikutanen oder intradermalen Applikation werden die aktiven Verbindungen oder deren physiologisch verträgliche Salze, gewünschtenfalls mit den pharmazeutisch üblichen Hilfsstoffen, beispielsweise zur Isotonierung oder pH-Einstellung sowie Lösungsvermittler, Emulgatoren, oder anderen Hilfsstoffen, in Lösung, Suspension oder Emulsion gebracht.

Aufgrund der kurzen Halbwertszeiten einiger der beschriebenen Arzneistoffe in Körperflüssigkeiten ist der Einsatz von injizierbaren Retardzubereitungen sinnvoll. Als Arzneiformen können z. B. ölige Kristallsuspensionen, Mikrokapseln, Rods oder Implantate verwendet werden, wobei die letzteren aus gewebeverträglichen Polymeren, insbesondere bioabbaubaren Polymeren, wie z. B. auf der Basis von Polymilchsäure-Polyglykolsäure-Copolymeren oder Humanalbumin aufgebaut sein können.

Eine besondere Bedeutung besitzt für die erfindungsgemäß zu verwendenden Kombinationspräparate die topische Verabreichung.

Die wirksame Dosis beträgt mindestens 0.001 mg/kg/Tag, vorzugsweise mindestens 0.01 mg/kg/Tag, insbesondere mindestens 0,1 mg/kg/Tag bis höchstens 3 mg/kg/Tag, vorzugsweise bis höchstens 1 mg/Tag/kg Körpergewicht, bezogen auf einen Erwachsenen von 75 kg Gewicht. Das weitere Antivirusmittel wird ggf. vorzugsweise in seinem bekannten Dosierungsbereich eingesetzt.

Das Mengenverhältnis von Bradykinin-Antagonist zu weiterem Antivirusmittel kann einen weiten Bereich überstreichen. Bevorzugt ist ein Verhältnis von 1:100 bis 100:1.

Durch das nachfolgende Ausführungsbeispiel sowie durch den Inhalt der Patentansprüche soll die vorliegende Erfindung näher erläutert werden.

### Beispiel 1. Vergleichendes Beispiel

### Prüfung der Wirkung von HOE 140 auf HSV-1 bedingten Hautläsionen bei haarlosen Mäusen

### Durchführung:

Zur Prüfung der antiviralen Wirksamkeit wurden immunkompetente haarlose Mäuse (hr/hr) mit Hilfe eines Glasfaserstiftes an einer Körperseite skarifiziert und durch Einreiben mit einer Virussuspension (HSV-1, klinisches Isolat "corneae") in einer Verdünnung 1/100 infiziert. Die infizierten Tiere wurden den verschiedenen Versuchgruppen zugeteilt und einzeln in Makrolonkäfigen gehalten.
Für die topische Behandlung wurde das Präparat in den unten angegebenen Konzentrationen in eine neutrale Cremezubereitung (Öl-in-Wasser-Emulsion) eingearbeitet. Die Behandlung erfolgte durch Einreiben der infizierten Stelle mit ca. 50 mg Creme 2 x täglich von 4. bis 14. Tag nach Infektion.
Bei der systemischen Behandlung wurde das Präparat in 0.9 % NaCl gelöst und 2 x täglich vom 4. bis 14. Tag nach Infektion subcutan verabreicht. Als Bewertungskriterien wurden die Schwere der virusbedingten Hautläsionen, sowie die Anzahl der Überlebenden und die mittlere Überlebendsdauer der verstorbenen Tiere herangezogen.

### Ergebnis:

Die Ergebnisse sind in folgender Tabelle zusammengefaßt:

Prüfung der Wirkung von HOE 140 auf HSV-1 bedingte Hautläsionen bei haarlosen Mäusen (12/93). Behandlung 2 x täglich über 10 Tage, beginnend 4 Tage nach kutaner Infektion der skarifizierten Rückenhaut.

| Dosis | Überlebende/Gruppengröße | mittlere Überlebenszeit (Tage) | Tiere mit Gürtelbildung |
|---|---|---|---|
| 1 % topisch | 3/5 | 9.0 ± 1.4 | 1 |
| 5 % topisch | 2/5 | 9.0 ± 2.0 | 2 |
| 1 mg/kg s.c. | 3/5 | 8.0 ± 0 | 1 |
| 5 mg/kg s.c. | 2/5 | 8.0 ± 0 | 0 |
| Kontrolle | 1/6 | 9.3 ± 1.5 | 5 |

Die Ausbildung schwerer Hautläsionen (Gürtelbildung) war bei den behandelten Tieren signifikant (p < 0.001, Chi²-Test) geringer als bei den unbehandelten Kontrollen, während die Überlebensrate weniger beeinflußt wurde (nicht signifikant, Chi²-Test).
Die vorliegenden Versuche zeigen, daß sowohl die parenterale (s.c.) als auch topische Behandlung mit HOE 140 eine deutliche Minderung der Symptome einer kutanen Herpesinfektion bewirkt.

### Vier-Felder-Test

| Vorgabe | Ergebnis | | Summe |
|---|---|---|---|
| | positiv | negativ | |
| Behandlung | 16 | 4 | 20 |
| Kontrolle | 1 | 5 | 6 |
| | | | |
| Summe | 17 | 9 | 26 |

| | CHI² 8.18 | | |
|---|---|---|---|
| Überschreitungswahrscheinlichkeit (P %) | 5 | 1 | 0.1 |
| CHI² Verteilung, zweiseitiger Test | 3.84 | 6.63 | 10.83 |
| einseitiger Test | 1.92 | 3.32 | 5.42 |

### Beispiel 2

Prüfung der Wirkung von HOE 140 auf HSV-1 bedingte Hautläsionen bei haarlosen Mäusen in Kombination mit Aciclovir.

HOE 140 wurde 2 x täglich entweder topisch als 2 %ige Creme in einer neutralen Grundlage oder subcutan mit 2 mg/kg pro Dosierung verabreicht, Aciclovir wurde als 0,05 %ige Lösung im Trinkwasser verabreicht. Um eine Überlegenheit der Kombinationstherapie nachzuweisen, wurden Infektionsstärke und Dosierung der Substanzen so gewählt, daß die Therapie mit den Einzelsubstanzen suboptimal ausfiel.
- Versuch 22/94:: Verabreichung Aciclovir Tag 0 - 7
Verabreichung HOE 140 Tag 3 - 8

| Dosis HOE 140 topisch oder s.c. | Aciclovir oral | Überlebende/Gruppengröße | mittlere Überlebenszeit (Tage) | Tiere mit Gürtelbildung |
|---|---|---|---|---|
| 12 x 2 % | | 2/8 | 8,3 ± 1,0 | 7 |
| 12 x 2 mg/kg s.c. | | 3/8 | 9,4 ± 1,3 | 5 |
| | | | | |
| 12 x 2 % | 0,05 % | 5/8 | 10,0 ± 1,7 | 3 |
| 12 x 2 mg/kg s.c. | 0,05 % | 8 ∗∗ | | 0 |
| | | | | |
| 12 x 0 % | | 0/8 | 9,6 ± 1,2 | 4 |

| | | | | |
|---|---|---|---|---|
| ∗∗) p (Chi²-Test) < 0,01 gegenüber Kontrolle | | | | |

Die Mortalitätsrate, sowie die Bildung schwerer Hautläsionen (Gürtelbildung) waren bei der Kombinationstherapie vermindert.
- Versuch 24/94:: Verabreichung Aciclovir Tag 3- 7
Verabreichung HOE 140 Tag 3- 7 oder 0 - 7

| Dosis HOE 140 topisch oder s.c. | Aciclovir oral | Überlebende/Gruppengröße | mittlere Überlebenszeit (Tage) | Tiere mit Gürtelbildung |
|---|---|---|---|---|
| 10 x 2 | - | 0/8∗ | 7,8 ± 0,5 ¹ | 8 |
| - | 0,05 % | 2/8∗ | 7,3 ± 1,2 | 7 |
| | | | | |
| 10 x 2 | 0,05 % | 3/8∗ | 9,0 ± 0,7 ^{1,}² | 8 |
| 16 x 2 | 0,05 % | 1/8∗ | 8,6 ± 1,0 ^{1,}² | 8 |
| | | | | |
| 10 x 0 % | - | 0/9 | 7,1 ± 0,9 | 9 |

| | | | | |
|---|---|---|---|---|
| ¹⁾ p (t-Test) < 0,05 gegenüber Kontrolle | | | | |
| ²⁾ p (t-Test) < 0,05 gegenüber Aciclovir alleine | | | | |
| ^{∗)} p (chi²-Test) nicht signifikant | | | | |

In einem 2. Kombinationsversuch wurde bestätigt, daß die Kombination von HOE 140 mit Aciclovir einer Therapie mit HOE 140 oder Aciclovir unter den gleichen Versuchsbedingungen überlegen ist. Während in diesem Versuch keine signifikanten Unterschiede in der Überlebensrate auftraten, war die Überlebensdauer der Tiere bei der Kombinationstherapie gegenüber den anderen Therapieformen, sowie der unbehandelten Kontrolle signifikant erhöht.

## Patentansprüche

1. Verwendung eines Bradykinin-Antagonisten der Formel I in Kombination mit mindestens einem weiteren Antivirusmittel zur Herstellung eines Medikaments zur Prophylaxe oder zur Behandlung von Erkrankungen, die durch Herpesviren oder Varicella Zoster Viren hervorgerufen werden, wobei die Formel I wie folgt lautet
Z - P - A - B - C - E - F - K - (D)Q - G - M - F' - I (I),
in welcher bedeuten:
Z a₁) Wasserstoff, (C₁-C₈)-Alkyl, (C₁-C₈)-Alkanoyl, (C₁-C₈)-Alkoxycarbonyl, (C₃-C₈)-Cycloalkyl, (C₄-C₉)-Cycloalkanoyl, oder (C₁-C₈)-Alkylsulfonyl,
in welchen jeweils 1, 2 oder 3 Wasserstoffatome gegebenenfalls durch 1, 2 oder 3 gleiche oder verschiedene Reste aus der Reihe
Carboxy, NHR(1), [(C₁-C₄)-Alkyl]NR(1) oder [(C₆-C₁₀)-Aryl-(C₁-C₄)-alkyl]NR(1),
wobei R(1) für Wasserstoff oder eine Urethan-Schutzgruppe steht,
(C₁-C₄)-Alkyl, (C₁-C₈)-Alkylamino, (C₆-C₁₀)-Aryl-(C₁-C₄)-alkylamino, Hydroxy, (C₁-C₄)-Alkoxy, Halogen, Di-(C₁-C₈)-alkylamino, Di-[(C₆-C₁₀)-aryl-(C₁-C₄)]-alkylamino, Carbamoyl, Phthalimido, 1,8-Naphthalimido, Sulfamoyl, (C₁-C₄)-Alkoxycarbonyl, (C₆-C₁₄)-Aryl und (C₆-C₁₄)-Aryl-(C₁-C₅)-alkyl ersetzt sind,
oder in welchen jeweils 1 Wasserstoffatom gegebenenfalls durch einen Rest aus der Reihe
(C₃-C₈)-Cycloalkyl, (C₁-C₆)-Alkylsulfonyl, (C₁-C₆)-Alkylsulfinyl, (C₆-C₁₄)-Aryl-(C₁-C₄)-alkylsulfonyl, (C₆-C₁₄)-Aryl-(C₁-C₄)-alkylsulfinyl, (C₆-C₁₄)-Aryl, (C₆-C₁₄)-Aryloxy, (C₃-C₁₃)-Heteroaryl und (C₃-C₁₃)-Heteroaryloxy
und 1 oder 2 Wasserstoffatome durch 1 oder 2 gleiche oder verschiedene Reste aus der Reihe
Carboxy, Amino, (C₁-C₈)-Alkylamino, Hydroxy, (C₁-C₄)-Alkoxy, Halogen, Di-(C₁-C₈)-alkylamino, Carbamoyl, Sulfamoyl, (C₁-C₄)-Alkoxycarbonyl, (C₆-C₁₄)-Aryl und (C₆-C₁₄)-Aryl-(C₁-C₅)-alkyl ersetzt sind;
a₂) (C₆-C₁₄)-Aryl, (C₇-C₁₅)-Aroyl, (C₆-C₁₄)-Arylsulfonyl, (C₃-C₁₃)-Heteroaryl, oder (C₃-C₁₃)-Heteroaroyl;
a₃) Carbamoyl, das gegebenenfalls am Stickstoff durch (C₁-C₈)-Alkyl, (C₆-C₁₄)-Aryl oder (C₆-C₁₄)-Aryl-(C₁-C₅)-alkyl substituiert sein kann;
wobei in den unter a₁), a₂) und a₃) definierten Resten die Aryl-, Heteroaryl-, Aroyl-, Arylsulfonyl- und Heteroaroyl-Gruppen gegebenenfalls durch 1, 2, 3 oder 4 Reste aus der Reihe
Carboxy, Amino, Nitro, (C₁-C₈)-Alkylamino, Hydroxy, (C₁-C₆)-Alkyl, (C₁-C₆)-Alkoxy, (C₆-C₁₄)-Aryl, (C₇-C₁₅)-Aroyl, Halogen, Cyano, Di-(C₁-C₈)-alkylamino, Carbamoyl, Sulfamoyl und (C₁-C₆)-Alkoxycarbonyl substituiert ist;
P für eine direkte Bindung steht oder einen Rest der Formel II bedeutet,
- NR(2) -(U)- CO - (II)
worin
R(2) Wasserstoff, Methyl oder eine Urethan-Schutzgruppe bedeutet,
U (C₃-C₈)-Cycloalkyliden, (C₆-C₁₄)-Aryliden, (C₃-C₁₃)-Heteroaryliden, (C₆-C₁₄)-Aryl-(C₁-C₆)-alkyliden, welche gegebenenfalls substituiert sein können, oder [CHR(3)]ₙ bedeutet,
wobei n 1 - 8, vorzugsweise 1 - 6 ist,
R(3) unabhängig voneinander Wasserstoff, (C₁-C₆)-Alkyl, (C₃-C₈)-Cycloalkyl, (C₆-C₁₄)-Aryl, (C₃-C₁₃)-Heteroaryl, bedeutet, die mit Ausnahme des Wasserstoffs jeweils gegebenenfalls durch
Amino, substituiertes Amino, Amidino, substituiertes Amidino, Hydroxy, Carboxy, Carbamoyl, Guanidino, substituiertes Guanidino, Ureido, substituiertes Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 1,8-Naphthalimido, 4-Imidazolyl, 3-Indolyl, 2-Thienyl, 3-Thienyl, 2-Pyridyl, 3-Pyridyl oder Cyclohexyl monosubstituiert sind,
wobei substituiertes Amino bevorzugt für -N(A')-Z, substituiertes Amidino bevorzugt für -(NH=)C-NH-Z, substituiertes Guanidino bevorzugt für -N(A')-C[=N(A')]-NH-Z und substituiertes Ureido bevorzugt für -CO-N(A')-Z stehen, in denen A' unabhängig voneinander Wasserstoff oder Z bedeutet, wobei Z wie unter a₁) oder a₂) definiert ist;
oder
worin R(2) und R(3) zusammen mit den diese tragenden Atomen ein mono-, bi- oder tricyclisches Ringsystem mit 2 bis 15 C-Atomen bilden;
A wie P definiert ist;
B eine basische Aminosäure in der L- oder D-Konfiguration, die in der Seitenkette substituiert sein kann;
C eine Verbindung der Formel III a oder III b
G'-G'-Gly (III a)
G'-NH-(CH₂)ₚ-CO (III b)
worin
p 2 bis 8 ist, und
G' unabhängig voneinander einen Rest der Formel IV
-NR(4)-CHR(5)-CO- (IV)
worin
R(4) und R(5) zusammen mit den diese tragenden Atomen ein heterocyclisches mono-, bi- oder tricyclisches Ringsystem mit 2 bis 15 C-Atomen bilden;
E den Rest einer neutralen, sauren oder basischen, aliphatischen oder alicyclisch-aliphatischen Aminosäure;
F unabhängig voneinander den Rest einer neutralen, sauren oder basischen, aliphatischen oder aromatischen Aminosäure, die in der Seitenkette substituiert sein kann, oder für eine direkte Bindung;
(D)Q D-Tic, D-Phe, D-Dic, D-Thi oder D-Nal, die gegebenenfalls durch Halogen, Methyl oder Methoxy substituiert sein können oder einen Rest der nachstehenden Formel (V) 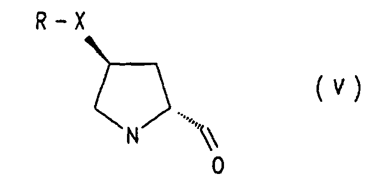 worin
X für Sauerstoff, Schwefel oder eine direkte Bindung steht;
R Wasserstoff, (C₁-C₈)-Alkyl, (C₃-C₈)-Cycloalkyl, (C₆-C₁₄)-Aryl, (C₆-C₁₄)-Aryl-(C₁-C₄)-alkyl, wobei der Alicyclus gegebenenfalls durch Halogen, Methyl oder Methoxy substituiert sein kann;
G wie G' oben definiert ist oder eine direkte Bindung;
F' wie F definiert ist, oder einen Rest -NH-(CH₂)_{q}-, mit q = 2 bis 8, oder, falls G keine direkte Bindung bedeutet, eine direkte Bindung;
I -OH, -NH₂ oder NHC₂H₅;
K den Rest -NH-(CH₂)ₓ-CO- mit x = 1 - 4, oder eine direkte Bindung, und
M wie F definiert ist,
oder eines seiner physiologisch verträglichen Salze.

2. Verwendung nach Anspruch 1 eines Bradykinin-Antagonisten der Formel I, in welcher bedeuten:
Z Wasserstoff oder wie unter a₁), a₂) oder a₃) definiert,
P eine Bindung oder einen Rest der Formel II
- NR(2) -(U)- CO - (II)
mit
U gleich CHR(3) und
R(3) wie oben defininert,
R(2) gleich H oder CH₃,
A eine Bindung.

3. Verwendung nach Anspruch 1 eines Bradykinin-Antagonisten der Formel I, in der bedeuten:
Z Wasserstoff oder wie unter a₁), a₂) oder a₃) definiert,
P eine Bindung oder ein Rest der Formel II
- NR(2) -(U)- CO - (II)
mit
U gleich CHR(3) und
mit R(3) unabhängig voneinander Wasserstoff, (C₁-C₆)-Alkyl, (C₃-C₈)-Cycloalkyl, (C₆-C₁₄)-Aryl, (C₃-C₁₃)-Heteroaryl, die mit Ausnahme des Wasserstoffs jeweils gegebenenfalls durch
Amino; substituiertes Amino, Hydroxy, Carboxy, Carbamoyl, Guanidino, substituiertes Guanidino, Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 4-Imidazolyl, 3-Indolyl, 2-Thienyl, 3-Thienyl, 2-Pyridyl, 3-Pyridyl oder Cyclohexyl monosubstituiert sind,
wobei substituiertes Amino bevorzugt für -N(A')-Z und substituiertes Guanidino bevorzugt für -N(A')-C[=N(A')]-NH-Z stehen, in denen A' unabhängig voneinander Wasserstoff oder Z bedeutet, wobei Z wie unter a₁) oder a₂) definiert ist;
oder
worin R(2) und R(3) zusammen mit den diese tragenden Atomen ein mono-, bi- oder tricyclisches Ringsystem mit 2 bis 15 C-Atomen bilden;
R(2) gleich H oder CH₃,
A eine Bindung,
(D)Q D-Tic.

4. Verwendung eines Bradykinin-Antagonisten zur Herstellung eines Medikaments zur Prophylaxe oder zur Behandlung von Viruserkrankungen nach Anspruch 1, **dadurch gekennzeichnet, daß** der Bradykinin-Antagonist (R)-Arginyl-(S)-arginyl-(S)-prolyl-(2S,4R)-hydroxyprolyl)glycyl-(S)-[3-(2-thienyl)alanyl]-(S)-seryl-(R)-[(1,2,3,4-tetrahydro-3-isochinolyl)carbonyl]-(2S,3aS,7aS)-[(hexahydro-2-indolinyl)carbonyl]-(S)-arginin, N-Acetat ist.

## Claims

1. The use of a bradykinin antagonist of the formula I in combination with at least one other antiviral agent for the preparation of a medicament for the prophylaxis or treatment of diseases which are caused by herpes viruses or Varicella Zoster viruses, where formula I reads as follows:
Z - P - A - B - C - E - F - K - (D)Q - G - M - F' - I (I)
in which:
Z is a₁) hydrogen, (C₁-C₈)-alkyl, (C₁-C₈)-alkanoyl, (C₁-C₈)-alkoxycarbonyl, (C₃-C₈)-cycloalkyl, (C₄-C₉)-cycloalkanoyl, or (C₁-C₈)-alkylsulfonyl,
in which in each case 1, 2 or 3 hydrogen atoms are optionally replaced by 1, 2 or 3 identical or different radicals from the series comprising
carboxyl, NHR(1), [(C₁-C₄)-alkyl]-NR(1) or [(C₆-C₁₀)-aryl-(C₁-C₄)-alkyl]NR(1),
in which R(1) is hydrogen or a urethane protective group,
(C₁-C₄)-alkyl, (C₁-C₈)-alkylamino, (C₆-C₁₀)-aryl-(C₁-C₄)-alkylamino, hydroxyl, (C₁-C₄)-alkoxy, halogen, di- (C₁-C₈)-alkylamino, di- [(C₆-C₁₀)-aryl-(C₁-C₄)]-alkylamino, carbamoyl, phthalimido, 1,8-naphthalimido, sulfamoyl, (C₁-C₄)-alkoxycarbonyl; (C₆-C₁₄)-aryl and (C₆-C₁₄)-aryl-(C₁-C₅)-alkyl,
or in which in each case 1 hydrogen atom is optionally replaced by a radical from the series comprising
(C₃-C₈)-cycloalkyl, (C₁-C₆)-alkylsulfonyl, (C₁-C₆)-alkylsulfinyl, (C₆-C₁₄)-aryl-(C₁-C₄)-alkylsulfonyl, (C₆-C₁₄)-aryl-(C₁-C₄)-alkylsulfinyl, (C₆-C₁₄)-aryl, (C₆-C₁₄)-aryloxy, (C₃-C₁₃)-heteroaryl and (C₃-C₁₃)-heteroaryloxy
and 1 or 2 hydrogen atoms are replaced by 1 or 2 identical or different radicals from the series comprising
carboxyl, amino, (C₁-C₈)-alkylamino, hydroxyl, (C₁-C₄)-alkoxy, halogen, di-(C₁-C₈)-alkylamino, carbamoyl, sulfamoyl, (C₁-C₄)-alkoxycarbonyl, (C₆-C₁₄)-aryl and (C₆-C₁₄)-aryl-(C₁-C₅)-alkyl;
a₂) (C₆-C₁₄)-aryl, (C₇-C₁₅)-aroyl, (C₆-C₁₄)-arylsulfonyl, (C₃-C₁₃)-heteroaryl or (C₃-C₁₃)-heteroaroyl;
a₃) carbamoyl, which can optionally be substituted on the nitrogen by
(C₁-C₈)-alkyl, (C₆-C₁₄)-aryl or (C₆-C₁₄)-aryl- (C₁-C₅)-alkyl;
and in which, in the radicals defined under a₁), a₂) and a₃), the aryl, heteroaryl, aroyl, arylsulfonyl and heteroaroyl groups are optionally substituted by 1, 2, 3 or 4 radicals from the series comprising
carboxyl, amino, nitro, (C₁-C₈)-alkylamino, hydroxyl, (C₁-C₆)-alkyl, (C₁-C₆)-alkoxy, (C₆-C₁₄)-aryl, (C₇-C₁₅)-aroyl, halogen, cyano, di-(C₁-C₈)-alkylamino, carbamoyl, sulfamoyl and (C₁-C₆)-alkoxycarbonyl;
P is a direct bond or is a radical of the formula II
-NR(2)-(U)-CO- (II)
in which
R(2) is hydrogen, methyl or a urethane protective group,
U is (C₃-C₈)-cycloalkylidene, (C₆-C₁₄)-arylidene, (C₃-C₁₃)-heteroarylidene or (C₆-C₁₄)-aryl-(C₁-C₆)-alkylidene, which can optionally be substituted, or [CHR(3)]ₙ,
in which n is 1 - 8, preferably 1 - 6, the radicals R(3) independently of one another are hydrogen, (C₁-C₆)-alkyl, (C₃-C₈)-cycloalkyl, (C₆-C₁₄)-aryl or (C₃-C₁₃)-heteroaryl , which, with the exception of the hydrogen, are in each case optionally monosubstituted by
amino, substituted amino, amidino, substituted amidino, hydroxyl, carboxyl, carbamoyl, guanidino, substituted guanidino, ureido, substituted ureido, mercapto, methylmercapto, phenyl, 4-chlorophenyl, .4-fluorophenyl, 4-nitrophenyl, 4-methoxyphenyl, 4-hydroxyphenyl, phthalimido, 1,8-naphthalimido, 4-imidazolyl, 3-indolyl, 2-thienyl, 3-thienyl, 2-pyridyl, 3-pyridyl or cyclohexyl,
in which substituted amino is preferably -N(A')-Z, substituted amidino is preferably -(NH=)C-NH-Z, substituted guanidino is preferably -N(A')-C[=N(A')]-NH-Z and substituted ureido is preferably -CO-N(A')-Z, in which the radicals A' independently of one another are hydrogen or Z, in which Z is as defined under a₁) or a₂);
or
in which R(2) and R(3), together with the atoms carrying these, form a mono-, bi- or tricyclic ring system having 2 to 15 carbon atoms;
A is as defined for P;
B is a basic amino acid in the L- or D-configuration,
which can be substituted in the side chain;
C is a compound of the formula IIIa or IIIb
G'-G'-Gly (IIIa)
G'-NH-(CH₂)ₚ-CO (IIIb)
in which
p is 2 to 8 and
the radicals G' independently of one another are a radical of the formula IV
-NR(4)-CHR(5)-CO- (IV)
in which
R(4) and R(5), together with the atoms carrying these, form a heterocyclic mono-, bi- or tricyclic ring system having 2 to 15 carbon atoms;
E is the radical of a neutral, acid or basic, aliphatic or alicyclic-aliphatic amino acid;
the radicals F independently of one another are the radical of a neutral, acid or basic, aliphatic or aromatic amino acid, which can be substituted in the side chain, or a direct bond;
(D) Q is D-Tic, D-Phe, D-Dic, D-Thi or D-Nal, which can optionally be substituted by halogen, methyl or methoxy, or a radical of the following formula (V) 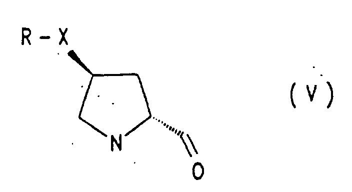 in which
X is oxygen, sulfur or a direct bond;
R is hydrogen, (C₁-C₈)-alkyl, (C₃-C₈)-cycloalkyl, (C₆-C₁₄)-aryl or (C₆-C₁₄)-aryl-(C₁-C₄)-alkyl, in which the alicyclic radical can optionally be substituted by halogen, methyl or methoxy;
G is as defined above for G' or a direct bond;
F' is as defined for F, a radical -NH-(CH₂)_{q}-, where q = 2 to 8, or, if G is not a direct bond, a direct bond;
I is -OH, -NH₂ or NHC₂H₅;
K is the radical -NH-(CH₂)ₓ-CO- where x = 1 to 4, or a direct bond and
M is as defined for F,
or one of its physiologically tolerated salts.

2. The use as claimed in claim 1 of a bradykinin antagonist of the formula I in which:
Z is hydrogen or is as defined under a₁), a₂) or a₃),
P is a bond or a radical of the formula II
-NR(2)-(U)-CO- (II)
where U is CHR(3) and
R(3) is as defined above,
R(2) is H or CH₃,
A is a bond.

3. The use as claimed in claim 1 of a bradykinin antagonist of the formula I in which:
Z is hydrogen or is as defined under a₁), a₂) or a₃),
P is a bond or a radical of the formula II
-NR(2)-(U)-CO- (II)
where U is CHR(3) and
where the radicals R(3) independently of one another are hydrogen, (C₁-C₆)-alkyl, (C₃-C₈)-cycloalkyl, (C₆-C₁₄)-aryl or (C₃-C₁₃)-heteroaryl, which, with the exception of the hydrogen, are in each case optionally monosubstituted by
amino, substituted amino, hydroxyl, carboxyl, carbamoyl, guanidino, substituted guanidino, ureido, mercapto, methylmercapto, phenyl, 4-chlorophenyl, 4-fluorophenyl, 4-nitrophenyl, 4-methoxyphenyl, 4-hydroxyphenyl, phthalimido, 4-imidazolyl, 3-indolyl, 2-thienyl, 3-thienyl, 2-pyridyl, 3-pyridyl or cyclohexyl,
and in which substituted amino is preferably -N(A')-Z and substituted guanidino is preferably -N(A')-C[=N(A')]-NH-Z, in which the radicals A' independently of one.another are hydrogen or Z, in which Z is as defined under a₁) or a₂);
or
in which R(2) and R(3), together with the atoms carrying these, form a mono-, bi- or tricyclic ring system having 2 to 15 carbon atoms;
R(2) is H or CH₃,
A is a bond,
(D)Q is D-Tic.

4. The use of a bradykinin antagonist for the prepara-. tion of a medicament for the prophylaxis or treatment of virus diseases as claimed in claim 1, wherein the bradykinin antagonist is (R)-arginyl-(S)-arginyl-(S)-prolyl-(2S,4R)-hydroxy-prolyl)glycyl-(S)-[3-(2-thienyl)alanyl]-(S)-seryl-(R)-[(1,2,3,4-tetrahydro-3-isoquinolyl)carbonyl] (2S,3aS,7aS)-[(hexahydro-2-indolinyl)carbonyl]-(S)-arginine N-acetate.

## Revendications

1. Utilisation d'un antagoniste de bradykinine de formule I en combinaison avec au moins un autre agent antiviral pour la préparation d'un médicament pour la prévention ou le traitement de maladies, qui sont provoquées par des Herpèsvirus ou le virus varicella zoster, la formule I répondant à la formule suivante
Z - P - A - B - C - E - F - K - (D)Q - G - M - F' - I (I),
dans laquelle
Z représente
a₁) un atome d'hydrogène, un groupe alkyle en C₁-C₈, alcanoyle en C₁-C₈, (alkoxy en C₁-C₈)-carbonyle, cycloalkyle en C₃-C₈, cycloalcanoyle en C₄-C₉ ou (alkyl en C₁-C₈)-sulfonyle,
dans lesquels chaque fois 1, 2 ou 3 atomes d'hydrogène sont éventuellement remplacés par 1, 2 ou 3 restes identiques ou différents pris dans la série
carboxy, NHR(1), [(alkyl en C₁-C₄]-NR(1) ou [(aryl en C₆-C₁₀)-(alkyle en C₁-C₄]NR(1),
où R(1) représente un atome d'hydrogène ou un groupe protecteur d'uréthanne,
alkyle en C₁-C₄, (alkyl en C₁-C₈)amino, (aryl en C₆-C₁₀)-(alkyl en C₁-C₄)amino, hydroxy, alkoxy en C₁-C₄, halogène, di-(alkyl en C₁-C₈)amino, di-[(aryl en C₆-C₁₀)-(alkyl en C₁-C₄)]amino, carbamoyle, phtalimido, 1,8-naphtylimido, sulfamoyle, (alkoxy en C₁-C₄)-carbonyle, aryle en C₆-C₁₄ et (aryl en C₆-C₁₄)-(alkyle en C₁-C₅),
ou dans lesquels chaque fois 1 atome d'hydrogène est éventuellement remplacé par un reste pris dans la série
cycloalkyle en C₃-C₈, (alkyl en C₁-C₆)-sulfonyle, (alkyl en C₁-C₆)sulfinyle, (aryl en C₆-C₁₄)-(alkyl en C₁-C₄)-sulfonyle, (aryl en C₆-C₁₄)-(alkyl en C₁-C₄)sulfinyle, aryle en C₆-C₁₄, aryloxy en C₆-C₁₄, hétéroaryle en C₃-C₁₃ et hétéroaryloxy en C₃-C₁₃
et 1 ou 2 atomes d'hydrogène sont remplacés par 1 ou 2 restes identiques ou différents pris dans la série comprenant
carboxy, amino, (alkyl en C₁-C₈)amino, hydroxy, alkoxy en C₁-C₄, halogène, di (alkyl en C₁-C₈) amino, carbamoyle, sulfamoyle, (alkoxy en C₁-C₄)-carbonyle, aryle en C₆-C₁₄, (aryl en C₆-C₁₄)-(alkyle en C₁-C₅) ;
a₂) aryle en C₆-C₁₄, aroyle en C₇-C₁₅, (aryl en C₆-C₁₄)-sulfonyle, hétéroaryle en C₃-C₁₃ ou hétéroaroyle en C₃-C₁₃ ;
a₃) carbamoyle, qui peut éventuellement être substitué sur l'atome d'azote par un groupe alkyle en C₁-C₈, aryle en C₆-C₁₄ ou (aryl en C₆-C₁₄)-(alkyle en C₁-C₅) ;
dans les restes définis aux points a₁), a₂) et a₃) les groupes aryle, hétéroaryle, aroyle, arylsulfonyle et hétéroaroyle sont éventuellement substitués par 1, 2, 3 ou 4 restes pris dans la série
carboxy, amino, nitro, (alkyl en C₁-C₈)-amino, hydroxy, alkyle en C₁-C₆, alkoxy en C₁-C₆, aryle en C₆-C₁₄, aroyle en C₇-C₁₅, halogène, cyano, di (alkyl en C₁-C₈)-amino, carbamoyle, sulfamoyle et (alkoxy en C₁-C₆)carbonyle ;
P représentent une liaison directe ou un reste de formule II,
- NR(2) -(U)- CO - (II)
où
R(2) représente un atome d'hydrogène, un groupe méthyle ou un groupe protecteur d'uréthanne,
U représente un groupe cycloalkylidène en C₃-C₈, arylidène en C₆-C₁₄, hétéroarylidène en C₃-C₁₃, (aryl en C₆-C₁₄)-(alkylidène en C₁-C₆) ou [CHR(3)]ₙ,
n va de 1 à 8, de préférence de 1 à 6,
R(3) indépendamment les uns des autres sont un atome d'hydrogène, un groupe alkyle en C₁-C₆, cycloalkyle en C₃-C₈, aryle en C₆-C₁₄, hétéroaryle en C₃-C₁₃, qui sont éventuellement chacun, à l'exception de l'hydrogène, monosubstitué par des substituants
amino, amino substitué, amidino, amidono substitué, hydroxy, carboxy, carbamoyle, guanidino, guanidino substitué, uréido, uréido substitué, mercapto, méthylmercapto, phényle, 4-chlorophényle, 4-fluorophényle, 4-nitrophényle, 4-méthoxyphényle, 4-hydroxyphényle, phtalimido, 1,8-naphtalimido, 4-imidazolyle, 3-indolyle, 2-thiényle, 3-thiényle, 2-pyridyle, 3-pyridyle ou cyclohexyle,
amino substitué représente de préférence un groupe -N(A')-Z, amidino substitué représente de préférence -(NH=)C-NH-Z, guanidino substitué représente de préférence -N(A')-C[(=N(A')]-NH-Z et uréido substitué représente de préférence -CO-N(A')-Z, dans lesquelles A' indépendamment les uns des autres représentent un atome d'hydrogène ou Z, Z étant défini comme au point a₁) ou a₂) ;
ou
où R(2) et R(3) conjointement avec les atomes qui les portent forment un système cyclique mono-, bi- ou tricyclique présentant 2 à 15 atomes de carbone ;
A est défini comme P ;
B représente un acide aminé basique en configuration L ou D, qui peut être substitué dans la chaîne latérale ;
C représente un composé de formule IIIa ou IIIb
G'-G'-Gly (IIIa)
G'-NH-(CH₂)ₚ-CO (IIIb)
où
p va de 2 à 8, et
G' indépendamment l'un de l'autre représentent un reste de formule
-NR(4)-CHR(5)-CO- (IV)
où
R(4) et R(5) conjointement avec les atomes qui les portent forment un système hétérocyclique mono-, bi- ou tricyclique présentant 2 à 15 atomes de carbone ;
E représente le reste d'un acide aminé aliphatique ou alicyclique-aliphatique neutre, acide ou basique ;
F indépendamment l'un de l'autre représentent le reste d'un acide aminé aliphatique ou aromatique neutre, acide ou basique, qui peut être substitué dans la chaîne latérale, ou représente une liaison directe ;
(D)Q, D-Tic, D-Phe, D-Dic, D-Thi ou D-Nal qui peuvent éventuellement être substitués par des substituants halogène, méthyle ou méthoxy ou représentent un reste de formule suivante 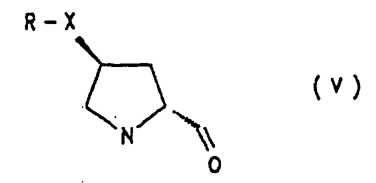 où
X représente un atome d'oxygène, un atome de soufre ou une liaison directe ;
R représente un atome d'hydrogène, un groupe alkyle en C₁-C₈, cycloalkyle en C₃-C₈, aryle en C₆-C₁₄ et (aryl en C₆-C₁₄)-(alkyle en C₁-C₄), l' alicycle peut éventuellement être substitué par des substituants halogène, méthyle ou méthoxy ;
G est défini comme G' ci-dessus ou représente une liaison directe ;
F' est défini comme F, ou représente un reste -NH-(CH₂)_{q}-, avec q = 2 à 8, ou, dans le cas où G ne n'est pas une liaison directe, représente une liaison directe ;
I représente -OH, -NH₂ ou NHC₂H₅ ;
K représente le reste -NH-(CH₂)ₓ-CO- avec x = 1-4 ou une liaison directe, et
M est défini comme F,
ou un de ses sels physiologiquement tolérés.

2. Utilisation selon la revendication 1, d'un antagoniste de bradykinine de formule I, dans laquelle représentent :
Z un atome d'hydrogène ou est défini comme aux points a₁), a₂) ou a₃),
P une liaison directe ou un reste de formule II,
- NR(2) -(U)- CO - (II)
avec
U égal à CHR(3) et
R(3). est défini comme ci-dessus,
R(2) un atome d'hydrogène ou un groupe CH₃,
A représente une liaison.

3. Utilisation selon la revendication 1 d'un antagoniste de bradykinine de formule I, dans laquelle représentent :
Z un atome d'hydrogène ou est défini comme aux points a₁), a₂) ou a₃),
P représente une liaison ou un reste de formule II
-NR(2)-(U)-CO- (II)
avec
U est CHR(3) et
R(3) indépendamment les uns des autres sont un atome d'hydrogène, un groupe alkyle en C₁-C₆, cycloalkyle C₃-C₈, aryle en C₆-C₁₄, hétéroaryle en C₃-C₁₃, à l'exception de l'atome d'hydrogène, chacun éventuellement monosubstitué par
amino, amino substitué, hydroxy, carboxy, carbamoyle, guanidino, guanidino substitué, uréido, mercapto, méthylmercapto, phényle, 4-chlorophényle, 9-fluorophényle, 4-nitrophényle, 4-méthoxyphényle, 4-hydroxyphényle, phtalimido, 4-imidazolyle, 3-indolyle, 2-thiényle, 3-thiényle, 2-pyridyle, 3-pyridyle ou cyclohexyle,
amino substitué représente de préférence -N(A')-Z et guanidino substitué représente de préférence -N(A')-C[(=N(A')]-NH-Z, dans lesquelles A' représentent, indépendamment les uns des autres, un atome d'hydrogène ou Z, Z étant défini comme au point a₁) ou a₂) ;
ou
où R(2) et R(3) conjointement avec les atomes qui les portent forment un système cyclique mono-, bi- ou tricyclique présentant 2 à 15 atomes de carbone ;
R(2) un atome d'hydrogène ou un groupe CH₃,
A représente une liaison,
(D)Q D-Tic.

4. Utilisation d'un antagoniste de bradykinine pour la préparation d'un médicament pour la prévention ou pour le traitement de maladies virales selon la revendication 1, **caractérisé en ce que** l'antagoniste de bradykinine est le N-acétate de (R)-arginyl-(S)-arginyl-(S)-prolyl-(2S,4R)-hydroxyprolyl)-glycyl-(S)-[3-(2-thiényl)alanyl]-(S)-séryl-(R)-[(1,2,3,4-tétrahydro-3-isoquinolyl)carbonyl]-2S,3aS,7aS)-[(hexahydro-2-indolinyl)carbonyl]-(S)-arginine.
